# FASCICULE DE BREVET EUROPEEN

(11) **EP 2 768 437 B1**
(45) Date de publication et mention de la délivrance du brevet: **24.02.2016**
(21) Numéro de dépôt: 12801617.7
(22) Date de dépôt: 16.10.2012
(51) Int. Cl.: A61F 2/46

(54) **PINCE DE PRÉHENSION D'IMPLANT INTERVERTÉBRAL, KIT ET ENSEMBLE DE MANIPULATION D'UNE TELLE PINCE.**
ZANGE ZUM FASSEN EINES BANDSCHEIBENIMPLANTATS, KIT UND ANORDNUNG ZUM MANÖVRIEREN EINER SOLCHEN ZANGE
FORCEPS FOR GRIPPING AN INTERVERTEBRAL IMPLANT, KIT AND ASSEMBLY FOR MANOEUVRING SUCH FORCEPS

(30) Priorité: 17.10.2011 FR 1159371
(43) Date de publication de la demande: 27.08.2014
(73) Titulaire: Les Laboratoires Osteal Medical, 95957 Roissy Aeroport CDG (FR)
(72) Inventeur: BOUCHOT, Daniel, F-77186 Noisiel (FR); HORYN, Yoachim, F-94160 Saint Mandé (FR); TISSERAND, Thierry, F-93600 Aulnay Sous Bois (FR)
(74) Mandataire: CAPRI
(86) Numéro de dépôt international: PCT/FR2012/052356
(87) Numéro de publication internationale: WO 2013/057420

(56) Documents cités:
- WO-A2-2005/051243
- WO-A2-2006/130460
- WO-A2-2008/021645
- US-A1- 2010 249 795

## Description

La présente invention concerne une pince de préhension d'implant intervertébral (ou prothèse de disque intervertébral) permettant de saisir de manière stable et unitaire un implant intervertébral destiné à être inséré entre deux vertèbres d'une colonne vertébrale en remplacement d'un disque intervertébral naturel détérioré. La présente invention concerne également un kit d'implantation comprenant une pince de préhension selon l'invention et un implant intervertébral en prise dans la pince. L'invention concerne en outre un ensemble d'implantation comprenant une pince de préhension selon l'invention, un implant intervertébral en prise dans la pince, et un ustensile de manipulation adapté à saisir la pince et à déplacer les mâchoires de la pince entre des positions fermée et ouverte. Le domaine d'application de la présente invention est bien entendu celui des accessoires chirurgicaux utilisés pour mettre en place un implant intervertébral entre deux vertèbres d'une colonne vertébrale.

On connaît le document WO2008/021654 qui décrit un instrument pour insérer un implant. En se référant à la figure 20 de ce document, on peut voir l'instrument dans une représentation explosée. L'implant est destiné à être saisi par deux mâchoires. Ces mâchoires sont montées sur deux bras comprenant chacun une surface de came inclinée. Les bras sont destinés à être engagés à l'intérieur d'une tige qui coulisse à l'intérieur d'un manchon. Ainsi, en rétractant la tige dans le manchon, les surfaces de came inclinées vont venir en prise avec le bord intérieur de l'extrémité ouverte du manchon, et contraindre ainsi les mâchoires en prise avec l'implant. Par conséquent, les surfaces de came n'ont pas pour fonction de contraindre les mâchoires en position ouverte, mais au contraire en position fermée. En effet, on ne voit pas de quelle manière les surfaces de came qui sont orientées vers l'extérieur pourraient être sollicitées de manière à contraindre les mâchoires en position ouverte.

Dans l'art antérieur, on connait déjà le document FR 2 893 839 qui décrit une instrumentation d'insertion d'un implant intervertébral entre deux vertèbres. Cette instrumentation d'insertion comprend une tige munie de deux pattes formant une pince et coulissant dans un tube. Le coulissement, en direction de l'extrémité de préhension, induit une fermeture de la pince par contact entre l'extérieur des pattes de la tige et une partie tronconique du tube, et le coulissement, en direction de l'extrémité de manipulation, induit une ouverture de la pince par contact entre un axe d'écartement du tube et l'intérieur des pattes de la tige. Les deux pattes sont reliées entre elles par un axe d'articulation qui est solidaire de la tige. Dans cette instrumentation d'insertion de l'art antérieur, la pince, formée par les deux pattes et son axe d'articulation, fait partie intégrante de l'instrumentation d'insertion, entre outre constituée par la tige, le tube et son axe d'écartement. De ce fait, il n'est pas possible de dissocier les pattes de la tige et ou du tube. D'autre part, il faut remarquer que la fermeture et le verrouillage de la pince s'effectue en rétractant les pattes à l'intérieur du tube, alors que l'ouverture de la pince s'effectue en étendant les pattes hors du tube contre l'axe d'écartement de ce dernier. La fermeture et l'ouverture de la pince s'effectuent donc avec des déplacements de la pince en sens inverse.

La présente invention a pour but de proposer une instrumentation d'insertion permettant de saisir une prothèse ou implant intervertébral sensiblement de la même manière que dans le document FR 2 893 839. Toutefois, l'instrumentation d'insertion de la présente invention présente une architecture différente de celle du document précité, qui permet un retrait ou remplacement aisé de la pince, qui présente en outre une configuration particulièrement simple, de sorte que la pince peut être à usage unique. Un autre but de la présente invention est de simplifier le mode opératoire de l'instrumentation d'insertion, et en particulier le mode de déplacement de la tige par rapport au tube. Encore un autre but de la présente invention est d'éliminer l'axe d'écartement de la pince, qui est à l'origine de l'architecture compliquée de l'instrumentation du document FR 2 893 839. En d'autres termes, la présente invention cherche à séparer la pince de l'ustensile de manipulation de la pince, de sorte que la pince peut être conditionnée sous la forme d'un kit avec l'implant intervertébral en prise dans la pince. Ainsi, la pince est associée à l'implant intervertébral et non pas à l'ustensile de manipulation de la pince, et peut de ce fait être à usage unique.

Pour atteindre ces buts, la présente invention propose une pince de préhension d'implant intervertébral comprenant deux bras rigides articulés l'un par rapport à l'autre au niveau d'un organe de connexion, les deux bras définissant entre eux un espace interne, chaque bras formant une mâchoire , les deux mâchoires étant destinées à venir en prise avec un implant intervertébral , les mâchoires étant mobiles entre une position ouverte et une position fermée en prise serrante avec l'implant, au moins un des deux bras comprenant une surface de came pour contraindre les mâchoires en position ouverte, caractérisée en ce que la surface de came s'étend à l'extérieur de l'espace interne défini entre les bras.

Dans le document FR 2 893 839 de l'art antérieur précité, les surfaces de cames sont formées par les faces internes des pattes alors que les profils de verrouillage sont formés par les faces extérieures des pattes. Dans la présente invention les surfaces de cames sont situées à l'extérieur des bras (ou pattes), de sorte que l'on peut éliminer l'axe d'écartement de la pince comme dans le document de l'art antérieur précité. Grâce au positionnement extérieur de la ou des surfaces de came par rapport au profil de verrouillage, il est possible de raccorder l'organe de connexion de la pince à la tige de l'ustensile de manipulation de manière très simple, étant donné qu'il n'est pas nécessaire de venir engager les bras de la pince autour d'un axe d'écartement. La présente invention peut donc être vue, selon un de ses aspects principaux, comme le déport de la ou des surfaces de came à l'extérieur des bras, en comparaison avec l'art antérieur précité du document FR 2 893 839.

Selon un aspect intéressant de la présente invention, ledit au moins un bras forme un logement externe situé à l'extérieur de l'espace interne, ce logement externe formant la surface de came. Le logement est donc situé sur le côté externe du bras, et non pas entre les bras.

Selon une caractéristique optionnelle de l'invention, un ou chaque bras comprend en outre un profil de verrouillage en position fermée. Avantageusement, le profil de verrouillage et la surface de came d'un bras sont orientés l'un vers l'autre. Cela ne signifie pas que la surface de came est disposée en face ou en regard direct du profil de verrouillage : cela signifie simplement que l'orientation générale de la surface de came est opposée à celle du profil de verrouillage de manière sensiblement convergente. Dans le document précité de l'art antérieur, la surface de came et le profil de verrouillage sont également orientés de manière opposée, mais de façon divergente.

Selon une autre caractéristique avantageuse de la présente invention, au moins un des deux bras comprend une branche et une patte qui s'étend environ parallèlement à la branche, la branche et la patte formant entre eux un logement externe ouvert en direction de l'organe de connexion, la patte formant la surface de came et la branche formant avantageusement un profil de verrouillage en position fermée. Ainsi, lorsque la pince comprend deux surfaces de came et deux profils de verrouillage, chaque paire formée par un profil de verrouillage et une surface de came est située dans un logement respectif, de sorte que chaque paire peut être actionnée par un organe de contact unique, comme on le verra ci-après.

Selon une forme de réalisation pratique, l'organe de connexion comprend des moyens de connexion amovibles adaptés à connecter la pince de préhension de manière amovible à un ustensile de manipulation adapté à saisir la pince et à déplacer ses mâchoires entre les positions fermée et ouverte. Ainsi, la pince ne fait pas partie intégrante de l'ustensile de manipulation, mais constitue un accessoire indépendant qui peut être combiné à l'implant pour constituer ensemble un kit d'implantation.

Selon un autre aspect de l'invention, la pince est réalisée de manière monobloc, les bras étant reliés à l'organe de connexion par une liaison souple. Ainsi, la pince de l'invention peut par exemple être réalisée par injection moulage d'une matière plastique appropriée. Elle peut également être réalisée par usinage ou encore par extrusion, par exemple de métal.

En résumé, dans une forme de réalisation préférentielle, la pince de préhension de l'invention comprend deux bras articulés formant chacun un logement tourné vers l'extérieur définissant une surface de came et avantageusement un profil de verrouillage. La pince de préhension est réalisée de manière monobloc et séparée de l'ustensile de manipulation, de sorte que la pince peut être associée à un implant intervertébral déjà en prise dans la pince, de manière à constituer un kit d'implantation qui peut être conditionné de manière stérile. De préférence, la pince est en position fermée à l'état de repos de manière à saisir l'implant intervertébral. Toutefois, il n'est pas exclus que la pince puisse se présenter en position ouverte en l'absence de contrainte extérieure.

La présente invention définit également un ensemble d'implantation comprenant une pince de préhension telle que définie ci-dessus, un implant intervertébral en prise dans la pince, et un ustensile de manipulation adapté à saisir la pince et à déplacer ses mâchoires entre les positions fermée et ouverte, cet ustensile de manipulation comprenant :
- un tube pourvu d'une poignée,
- une tige montée coulissante dans le tube selon un axe de coulissement X, la tige étant connectée à l'organe de connexion de la pince,
- un mécanisme pour déplacer la tige dans le tube selon l'axe de coulissement X,
caractérisé en ce que le tube de l'ustensile comprend au moins un organe de contact qui vient en prise avec la surface de came de la pince, lors du coulissement de la tige dans le tube. Lorsque la pince comprend deux surfaces de came, le tube forme deux organes de contact correspondant qui sont situés de part et d'autre de la tige, à l'extérieur de l'espace interne formé par la pince.

Selon une caractéristique intéressante de l'ensemble d'implantation, la tige comprend un profil de connexion connecté de manière amovible à l'organe de connexion de la pince, la tige coulisse dans le tube entre une position étendue dans laquelle l'organe de connexion de la pince est engageable sur le profil de connexion de la tige qui fait alors saillie hors du tube, et une position rétractée dans laquelle l'organe de contact est en prise avec la surface de came de la pince. La pince peut ainsi être facilement mise en place et retirée de l'ustensile, sans être gêné par le ou les organes de contact.

Selon une autre caractéristique de l'invention, la tige est déplaçable dans une position intermédiaire de verrouillage située entre les positions étendue et rétractée, dans laquelle l'organe de contact est alors en prise avec un profil de verrouillage formé par un bras de la pince. Ainsi, c'est un organe de contact unique qui actionne une paire constituée par un profil de verrouillage et une surface de came. Et comme la pince de préhension de l'invention comprend de préférence deux paires de profil de verrouillage/surface de came, il y a deux organes de contact. Il faut aussi remarquer qu'en rétractant la tige à l'intérieur du tube à partir de la position étendue, la tige passe par la position intermédiaire de verrouillage avant d'arriver à la position finale entièrement rétractée. En d'autres termes, la phase de montage de la pince sur l'ustensile, la phase de verrouillage de la pince en position fermée et la phase d'ouverture de la pince s'effectue en déplaçant la tige à l'intérieur du tube dans une seule et même direction. En pratique, l'ustensile de manipulation est initialement amené en position étendue de manière à ce que le kit d'implantation (pince de préhension plus implant intervertébral) puisse être connecté à l'ustensile de manipulation. Ensuite, l'utilisateur actionne le mécanisme de déplacement de la tige dans le tube de manière à rétracter la tige dans le tube jusqu'à la position intermédiaire de verrouillage. Dans cette position, la pince est en prise bloquée sur l'implant intervertébral, qui peut alors être introduit ou implanté entre deux vertèbres. Une fois cette opération d'implantation terminée, l'opérateur rétracte encore davantage la tige dans le tube de manière à atteindre la position rétractée dans laquelle la pince est contrainte en position ouverte. Elle peut alors être désolidarisée de l'implant intervertébral et retirée ensemble avec l'ustensile de manipulation.

Selon un autre aspect pratique de l'invention, l'ustensile comprend des moyens d'indication pour indiquer à l'utilisateur que la tige est en position intermédiaire de verrouillage. De cette manière, l'utilisateur de l'ustensile de manipulation peut savoir de manière garantie que la pince est verrouillée sur l'implant intervertébral.

Selon une autre caractéristique de l'invention, ledit au moins un organe de contact du tube se présente sous la forme d'une barrette dont la face externe vient en prise avec la surface de came et dont la face interne vient avantageusement en prise avec un profil de verrouillage du bras de la pince. C'est donc les deux faces d'un même organe qui vient en prise successive avec un profil de verrouillage et une surface de came.

Selon un autre aspect pratique de l'invention, l'organe de connexion de la pince est engagé latéralement ou axialement sur le profil de connexion de l'ustensile de manipulation en position étendue de la tige, la connexion entre l'organe de connexion et le profil de connexion étant verrouillée par l'introduction du profil de connexion dans le tube, le profil de connexion étant avantageusement déformable élastiquement pour garantir le maintien de l'organe de connexion sur le profil de connexion, même avant son introduction dans le tube. La mise en place de la pince sur l'ustensile est facilitée du fait qu'aucun élément de l'ustensile ne vient se positionner entre les bras de la pince. En effet, il n'y a pas d'axe d'écartement comme dans le document FR 2 893 839.

Un des principes de la présente invention réside dans le fait de disposer la ou les surface(s) de came de la pince à l'extérieur des bras, avantageusement à proximité d'un éventuel profil de verrouillage, de sorte qu'ils peuvent être tous deux actionnés par paire par un organe de contact unique. Le déplacement de la tige à l'intérieur du tube peut alors s'effectuer de manière unidirectionnelle à partir de la position étendue (mise en place de la pince) vers la position complètement rétractée (écartement des mâchoires) en passant optionnellement par une position intermédiaire de verrouillage.

L'invention sera maintenant plus amplement décrite en référence aux dessins joints, donnant à titre d'exemple non limitatif un mode de réalisation de l'invention.

Sur les figures :
La figure 1 est une vue de dessus d'une pince de préhension de l'invention en prise avec un implant intervertébral,
La figure 2 est une vue en perspective de la pince de préhension (et de l'implant) de la figure 1 connectée à un ustensile de manipulation de l'invention ;
Les figures 3a à 3d sont des vues en coupe horizontale à travers un implant, une pince et un ustensile de l'invention dans quatre configurations fonctionnelles permettant d'illustrer le mode opératoire de la pince et de l'ustensile de manipulation,
La figure 4 est une vue en perspective éclatée d'un ustensile de manipulation de l'invention prêt à être raccordé à un kit d'implantation formé par une pince de préhension de l'invention et un implant intervertébral, et
La figure 5 est une vue en coupe longitudinale à travers l'ustensile de manipulation de la figure 4 connecté à une pince de préhension de l'invention en prise avec un implant intervertébral.

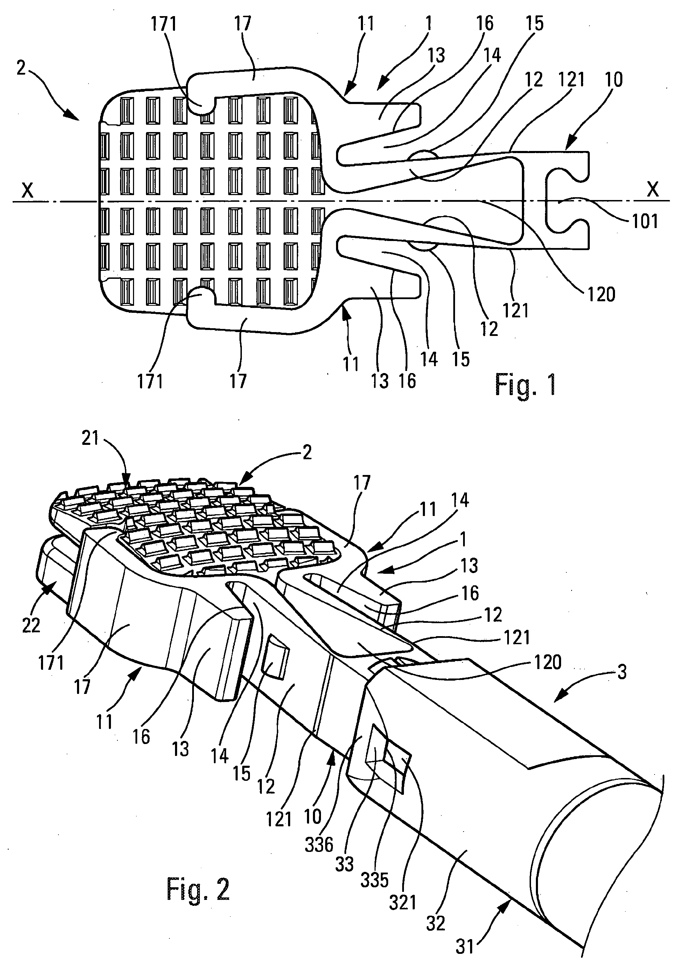

On se référera tout d'abord aux figures 1 et 2 pour décrire en détail la structure d'une pince de préhension selon l'invention. La pince, désignée dans son ensemble par la référence numérique 1, est de préférence réalisée de manière monobloc par injection/moulage de matière plastique, par usinage ou extrusion de métal, comme par exemple du titane pour l'usinage et l'aluminium pour l'extrusion. La pince 1 comprend deux bras 11 qui sont de préférence identiques par symétrie miroir matérialisée sur la figure 1 par l'axe X. Ces deux bras 11 sont raccordés à un organe de connexion 10 qui forme des moyens de connexion 101 sous la forme d'un logement de connexion. Dans l'exemple illustré sur les figures, ce logement de connexion est un logement femelle, mais un logement mâle conviendrait également. L'organe de connexion 10 est relié sur deux de ces bords opposés aux deux bras 11 par des liaisons élastiquement déformables 121, qui sont réalisées sous la forme d'une réduction d'épaisseur de paroi conférant une souplesse ou une résilience élastique à mémoire de forme. Ainsi, les deux bras 11 reviennent toujours dans leur position de repos initiale après avoir subi une contrainte. Sans sortir du cadre de l'invention, on peut également prévoir que les liaisons souples 121 ne soient pas élastiques, de sorte qu'après déformation, les bras 11 ne reviennent pas dans leur position initiale de repos. De préférence, les bras 11 sont rigides, de sorte que la seule déformation de la pince est localisée au niveau des liaisons souples 121. On peut aussi réaliser la pince avec un bras fixe et un bras mobile : dans ce cas, seul le bras mobile est relié à l'organe de connexion avec une liaison souple, le bras fixe étant fixement et solidement à l'organe de connexion.

Chaque bras 11 comprend une branche 12 qui est reliée à une de ses extrémités à l'organe de connexion 10 par la liaison souple 121. Les branches 12 s'étendent sensiblement parallèlement à l'axe X, en convergeant toutefois l'une vers l'autre en s'éloignant de l'organe de connexion 10. Elles pourraient également être parfaitement parallèles. Elles définissent entre elles un espace interne 120. A leurs autres extrémités, les branches 12 se prolongent en formant deux mâchoires 17 qui sont pourvues de dents (ou ergots) de préhension 171 destinées à venir en prise avec un implant intervertébral 2. L'implant 2 peut comprendre un plateau supérieur 21 et un plateau inférieur 22 qui sont déplaçables l'un par rapport sur un noyau, reproduisant ainsi les déplacements possibles de deux vertèbres séparées par un disque intervertébral naturel. Les mâchoires 17 de la pince sont dessinées pour recevoir de manière stable et fixe l'implant 2. Plus particulièrement, les dents de préhension 171 peuvent venir en prise avec le plateau supérieur 21 et le plateau inférieur 22 et les maintenir fixement l'un par rapport à l'autre. Les dents 171 peuvent par exemple s'étendre sur le plateau supérieur 21 et sous le plateau inférieur 22, ainsi qu'entre les deux plateaux pour les maintenir fermement.

Chaque bras 11 comprend également une patte (ou bride) 13 qui s'étend à partir de la mâchoire 17 sensiblement parallèlement à la branche 12, de manière à définir une extrémité libre orientée généralement vers l'organe de connexion 10. La patte 13 peut présenter une embase renforcée par rapport à son extrémité libre, de manière à présenter une forme trapézoïdale ou de coin. La patte 13 définit une face extérieure tournée en éloignement de la branche 12 et une face intérieure tournée vers la branche 12. Il est ainsi formé un logement externe 14 entre la branche 12 et la patte 13, qui est ouvert en direction de l'organe de connexion 10. Le logement 14 est situé à l'extérieur de l'espace interne 120 défini entre les deux branches 12. De préférence, les deux branches 11 sont formées avec une telle patte formant un logement externe 14. Toutefois, il n'est pas exclu qu'une pince selon l'invention ne soit formée qu'avec une seule patte 13 et un seul logement externe 14, l'autre patte en étant dépourvue. Dans le cas, l'ouverture de la pince s'effectue en ne déplaçant qu'une seule mâchoire. L'autre mâchoire peut être fixe.

Selon l'invention, la face intérieure de la patte 13 forme une surface de came 16 qui est inclinée par rapport à l'axe X de la pince 1. Plus précisément, la surface de came 16 se rapproche de l'axe X à mesure que l'on pénètre dans le logement externe 14. Ainsi, en engageant un organe de contact dans le logement externe 14 selon un axe parallèle à l'axe X, cet organe de contact va venir en contact de glissement contre la surface de came 16, ce qui va générer un déplacement du bras 11 par pivotement autour de la liaison souple 121. En résultat, les dents de préhension 171 vont se désengager de l'implant intervertébral 2. En prévoyant un organe de contact par logement externe 14, on peut déplacer ou contraindre les bras 11 de la pince dans une position ouverte permettant de retirer aisément la pince de l'implant 2. La pince 1 est de préférence en position fermée, tel que représenté sur la figure 1, à l'état de repos. Toutefois, on peut également envisager une pince qui soit ouverte à l'état de repos. Dans ce dernier cas, l'engagement d'organe de contact avec les surfaces de came 16 va simplement garantir l'ouverture de la pince au cas où les mâchoires 17 seraient entravées par un environnement quelconque. Toutefois, de préférence, la pince est en position fermée à l'état de repos, permettant ainsi de saisir l'implant intervertébral 2 sans avoir à exercer de contrainte externe sur la pince.

Selon une autre caractéristique intéressante de l'invention, les faces externes des bras 12 forment chacun un profil de verrouillage 15 sous la forme d'une protubérance ou saillie qui s'étend vers l'extérieur, sensiblement au niveau de l'extrémité libre des pattes 13. On peut ainsi considérer que les profils de verrouillage 15 sont situés dans les logements externes 14, ou directement au niveau de leur entrée. La fonction de ces profils de verrouillage 15 est de bloquer ou verrouiller la pince 1 en position fermée représentée sur les figures 1 et 2 à l'aide de l'organe de contact qui va ensuite venir en prise avec les surfaces de came 16. Les profils de verrouillage 15 peuvent aussi servir à déplacer les mâchoires en position fermée. Ces profils de verrouillage 15 constituent une caractéristique optionnelle dont on peut se passer, particulièrement dans le cas où la pince est en position fermée à l'état de repos. Il s'agit toutefois d'une caractéristique avantageuse qui garantit un verrouillage parfait de la pince en position fermée. Dans ce cas, on peut constater que pour un logement externe considéré, le profil de verrouillage 15 est orienté sensiblement vers la surface de came 16 de manière opposée. Il est également à noter que les profils de verrouillage 15, tout comme les surfaces de came 16, sont situés à l'extérieur de l'espace interne 120 défini entre les deux bras 11. Selon une autre définition possible, on peut dire que chaque logement externe 14 forme une surface de came 16 et optionnellement un profil de verrouillage 15. On peut également dire que les pattes ou brides 13 sont disposées de part et d'autre des branches 12 à l'extérieur du logement interne 120. Il s'agit là d'une caractéristique qui est particulièrement avantageuse, étant donné qu'il n'est pas nécessaire d'introduire un quelconque organe entre les deux branches 12 pour écarter les bras 11. Le déplacement des bras 11 de la position fermée vers la position ouverte est ainsi possible de l'extérieur des branches 12 par insertion d'un organe de contact dans les logements externes 14. On peut envisager de réaliser une pince avec un seul profil de verrouillage. Un bras peut être mobile, alors que l'autre est fixe. Le profil de verrouillage et la surface de came seraient alors prévus uniquement sur le bras mobile.

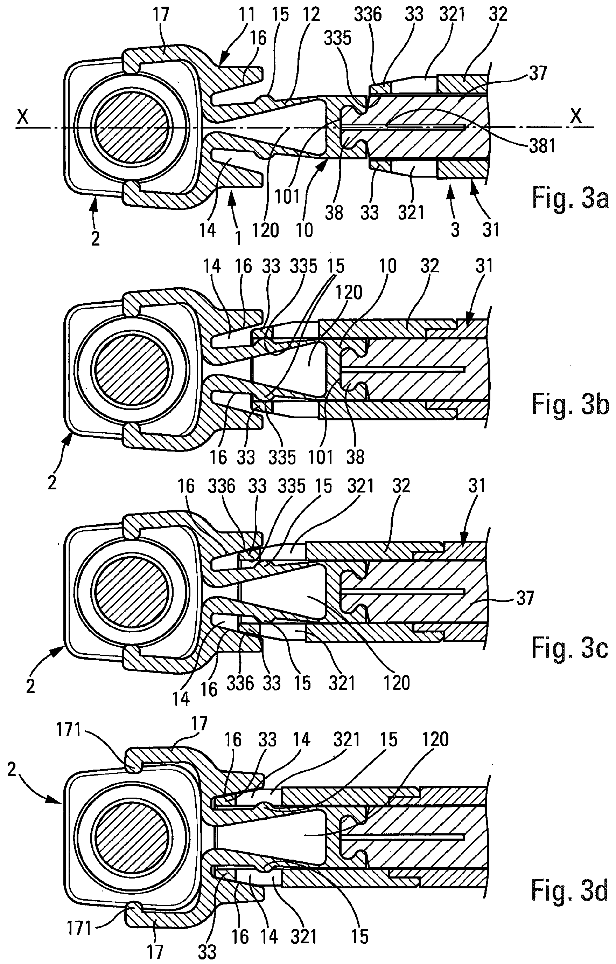

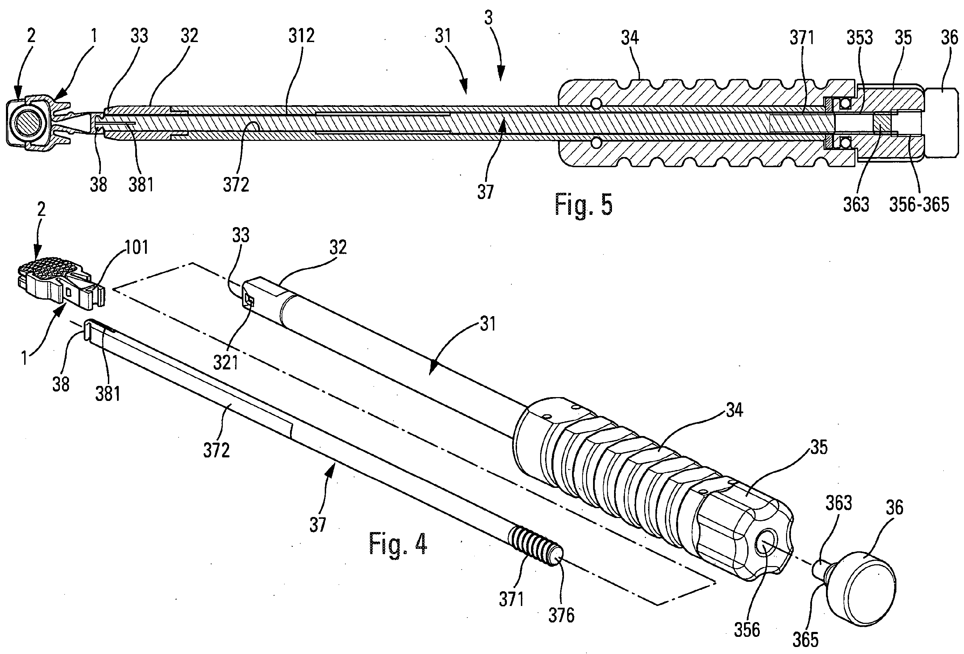

On se référera maintenant aux figures 3a à 3d qui illustrent de quelle manière la pince de préhension 1 peut être contrainte à l'état ouvert et optionnellement verrouillée à l'état fermé à l'aide d'un ustensile de manipulation 3 dont seule une extrémité est représentée. L'ustensile de manipulation 3 sera décrit ci-après de manière détaillée en référence aux figures 4 et 5. En résumé, l'ustensile de manipulation 3 comprend un tube creux 31 pourvu d'un embout 32 formant deux barrettes de contact 33 délimitées par deux fenêtres 321. L'ustensile de manipulation 3 comprend également une tige 37 qui est engagée à l'intérieur du tube 31 et de l'embout 32 de manière coulissante selon l'axe X, qui est confondu avec l'axe de symétrie miroir de la pince 1. L'extrémité de la tige 37 est formée avec un profil de connexion 38 qui est ici un profil mâle destiné à s'engager à l'intérieur du logement de connexion femelle 101 de l'organe de connexion 10 de la pince de préhension 1. A la place de cette tête de connexion mâle, on aurait également pu prévoir un logement de connexion femelle et dans ce cas l'organe de connexion 10 aurait été formé avec une tête de connexion mâle. Dans la configuration représentée sur les figures, le profil de connexion mâle 38 de la tige 37 est avantageusement formé avec une fente 381, lui conférant ainsi une certaine élasticité. De cette manière, on peut engager le profil de connexion 38 dans le logement femelle 101 de manière à assurer une certaine contrainte élastique du profil 38 à l'intérieur du logement 101. De cette manière, la pince peut être maintenue temporairement sur la tige 37. Cette étape d'engagement de la pince 1 sur la tige 37 est représentée sur la figure 3a. On peut voir que le profil de connexion mâle 38 de la tige 37 fait alors saillie vers l'extérieur au-delà de l'extrémité de l'embout 32 formée avec les deux barrettes 33. La mise en place de la pince sur la tige 37 s'effectue par un engagement latéral par rapport à l'axe X qui est également l'axe de coulissement de la tige 37 dans l'embout 32. On peut également envisager d'engager la pince axialement sur la tige avec un encliquetage. Une fois la pince 1 ainsi en place sur la tige 37, cette dernière peut être déplacée par coulissement vers l'intérieur du tube 31 et de l'embout 32, de sorte que le profil de connexion 38 et l'organe de connexion 10 pénètrent à l'intérieur de l'embout 32, comme visible sur la figure 3b. On comprend aisément que la connexion entre la pince 1 et la tige 37 est alors verrouillée par l'embout 32 qui les entoure. Il faut également remarquer que les deux barrettes 33 sont alors situées au niveau des deux profils de verrouillage 15, juste à l'entrée des logements externes 14, empêchant ainsi tout écartement des bras 11 de la pince. Plus précisément, chaque barrette 33 comprend une face interne 335 qui peut venir en contact avec la saillie formant le profil de verrouillage 15. On peut également remarquer que les deux barrettes 33 sont encore hors de contact des surfaces de came 16. Dans cette position intermédiaire, non seulement la pince est fixement et solidement arrimée à la tige 11, mais encore les deux bras 11 de la pince sont verrouillés en position fermée. On peut alors manipuler la pince 1 et son implant intervertébral 2 sans risque de perdre l'implant ou de rompre la connexion entre la pince et l'instrument de manipulation 3. L'ustensile de manipulation 3, la pince de préhension 1 et l'implant intervertébral 2 constituent alors un ensemble d'implantation unitaire qui peut être manipulé par un chirurgien de manière à mettre en place l'implant 2 entre deux vertèbres d'une colonne vertébrale. Une fois l'implant en place entre les deux vertèbres, il faut retirer la pince 1 de l'implant 2, et ceci se fait par écartement des deux mâchoires 17 des bras 11. Pour ce faire, on déplace encore davantage la tige 37 à l'intérieur du tube 31 de manière à ce que les barrettes 33 viennent en contact avec les surfaces de came 16 de la pince. Ceci est représenté sur la figure 3c. Plus précisément, chaque barrette 33 définit une face externe 336 qui vient en contact de glissement avec une surface de came respective 16. Etant donné que la tige 37 se déplace axialement selon l'axe X et que les surfaces de came 16 sont inclinées par rapport à ce même axe X, les faces externes 336 des barrettes 33 vont contraindre les bras 11 de la pince à s'écarter, à mesure que les barrettes se rapprochent du fond des logements externes 14. Cette position finale rétractée est représentée sur la figure 3d. Les dents de préhension 171 sont désengagées de l'implant intervertébral 2, et la pince 1 peut alors être retirée de l'implant qui reste en place entre les deux vertèbres. L'opération d'implantation proprement dite est alors terminée.

Les deux barrettes 33 constituent des organes de contact dont la fonction est de venir en prise avec la ou les surface(s) de came 16, et optionnellement avec les profils de verrouillage 15. Ainsi, un même organe de contact remplit une double fonction, à savoir celle d'écartement d'au moins un bras et celle de verrouillage des bras en position fermée. On peut remarquer que cet organe de contact est situé à l'extérieur de l'espace interne 120. Les différentes étapes de manipulation de la figure 3a à la figure 3d s'effectuent en déplaçant la tige 37 dans un seul et même sens, à savoir vers l'intérieur du tube 31. Il n'est donc pas nécessaire d'inverser le sens de déplacement de la tige 37 lors de l'implantation de l'implant intervertébral 2.

On peut également noter que les barrettes 33 ne sont jamais en contact simultanément avec les profils de verrouillage 15 et les surfaces de came 16. Ceci est visible sur la figure 3c. La barrette 33 passe successivement d'un contact avec les profils de came 15 à un contact avec les surfaces de came 16, évitant ainsi tout blocage ou durcissement dans la manipulation.

On se référera maintenant aux figures 4 et 5 pour décrire plus en détail l'ustensile de manipulation 3. Comme susmentionné, l'ustensile de manipulation 3 comprend une tige 37 qui est déplaçable par coulissement à l'intérieur d'un tube 31 pourvu à son extrémité d'un embout 32 formant les deux barrettes faisant office d'organe de contact. Pour déplacer la tige 37 à l'intérieur du tube 31, il est prévu un mécanisme qui permet de transformer un mouvement rotatif en un mouvement translatif, comme ceux utilisés dans les rouges à lèvres. Pour cela, le tube 31 est pourvu, à son extrémité opposée à l'embout 32, d'une mollette 35 qui est montée librement en rotation. Cette mollette 35 comprend intérieurement un premier filtrage 353 qui est en prise avec une extrémité filetée 371 de la tige 37 opposée au profil de connexion 38. D'autre part, la tige 37 est formée avec un ou deux méplat(s) 372 qui sont destinés à coulisser contre deux parois planes 312 formées à l'intérieur du tube 31. Ainsi, la tige 37 est bloquée en rotation à l'intérieur du tube 31. Et en actionnant la mollette 35, l'extrémité filetée 371 de la tige 37 est contrainte de se déplacer dans le filetage interne 353 de la mollette 35. Ainsi, le mouvement rotatif de la mollette 35 est transformé en un déplacement axial translatif de la tige 37 à l'intérieur du tube 31. Pour la prise en main du tube 31, il est avantageusement pourvu d'une poignée 34.

Selon une caractéristique avantageuse, la mollette 35 est pourvu d'une tête de butée 36 formant un pion de butée 363 et une section filetée 365. D'autre part, la mollette 35 comprend un second filetage interne 356 dans lequel le filetage 365 de la tête de butée 36 est vissé. Sur la figure 5, la tête de butée est vissée à fond dans la mollette 35 de sorte que le pion 363 est engagé à fond à l'intérieur de la mollette. On comprend alors aisément qu'en faisant tourner la mollette 35, la tige 37 va se déplacer à l'intérieur du tube 31 et de la mollette 35 jusqu'à venir en butée contre le pion 363 de la tête de butée 36. Cette position de butée correspond à la position intermédiaire de verrouillage représentée sur la figure 3c. En d'autres termes, lorsque la tête de butée 36 est vissée à fond sur la mollette 35, l'opérateur ou le chirurgien sait qu'en tournant la mollette 35 au maximum, il va atteindre la position intermédiaire de verrouillage, dans laquelle l'ensemble d'implantation peut être utilisé pour mettre en place l'implant intervertébral 2 entre deux vertèbres d'une colonne vertébrale. Une fois l'implant en place, l'opérateur ou le chirurgien dévisse la tête de butée 36 et tourne ensuite la mollette 35 de manière à parvenir jusqu'à la position rétractée de la figure 3d dans laquelle la pince 1 est en position ouverte. Ainsi, la tête de butée 36 sert de moyen d'indication physique permettant à l'utilisateur de savoir que l'ensemble d'implantation est en condition pour mettre en place l'implant 2 entre deux vertèbres. A la place ou en complément de la tête de butée 36, on peut également prévoir des indicateurs visuels comme par exemple un marquage au niveau de l'embout 32.

Le tube 31 est ici réalisé en deux pièces, mais il peut également être réalisé en une seule pièce, ou au contraire en trois pièces ou même davantage. Le blocage en rotation de la tige 37 dans le tube 31 est réalisé sur la longueur du tube 31, mais on peut également prévoir cette fonction de blocage uniquement au niveau de l'embout 32. L'embout 32 comprend deux barrettes de contact 33, mais on peut également prévoir uniquement une seule barrette dans le cas où seul un bras 11 est articulé. D'autre part, sans sortir du cadre de l'invention, on peut intervertir la surface de came et l'organe de contact, de sorte de la surface de came serait formée par le tube et l'organe de contact, qui vient en contact de glissement sur la surface de came, serait formé par la pince. Il ne s'agirait que d'une simple inversion de caractéristiques, sans modifier la fonction technique.

Le positionnement des surfaces de came 16 à l'extérieur des branches 12 permet de réaliser l'écartement des bras 11 sans avoir à accéder à l'espace interne 120 défini entre les deux bras 12. De ce fait, la mise en place et le retrait de la pince de préhension 1 sur l'ustensile de manipulation 3 est extrêmement aisé, étant donné que les organes de contact 33 sont totalement inopérants et retirés lors de la mise en place de la pince sur l'extrémité de la tige 37. La pince de préhension 1 constitue ainsi un élément séparable de l'ustensile de manipulation 3, de sorte que l'on peut l'associer à l'implant intervertébral 2 sous la forme d'un kit d'implantation, par exemple conditionné de manière stérile dans un emballage approprié. Une fois le kit mis en place sur l'ustensile de manipulation selon le mode opératoire précédemment décrit, l'ensemble constitue un ensemble d'implantation qui est manipulable de manière unitaire jusqu'à mise en place de l'implant intervertébral entre deux vertèbres. Ensuite, la pince peut être retirée de l'ustensile de manipulation 3 et mise au rebus en tant qu'accessoire à usage unique.

## Revendications

1. Pince de préhension (1) d'implant intervertébral (2) comprenant deux bras rigides (11) articulés l'un par rapport à l'autre au niveau d'un organe de connexion (10), les deux bras (11) définissant entre eux un espace interne (120), chaque bras (11) formant une mâchoire (17), les deux mâchoires (17) étant destinées à venir en prise avec un implant intervertébral (2), les mâchoires (17) étant mobiles entre une position ouverte et une position fermée en prise serrante avec l'implant (2), au moins un des deux bras (11) comprenant une surface de came (16) pour contraindre les mâchoires (17) en position ouverte,
**caractérisée en ce que** la surface de came (16) s'étend à l'extérieur de l'espace interne (120) défini entre les bras (11).

2. Pince de préhension (1) selon la revendication 1, dans laquelle ledit au moins un bras (11) forme un logement externe (14) situé à l'extérieur de l'espace interne (120), ce logement externe (14) formant la surface de came (16).

3. Pince de préhension (1) selon la revendication 1 ou 2, dans laquelle au moins un des deux bras (11) comprend un profil de verrouillage (15) en position fermée.

4. Pince de préhension (1) selon la revendication 3, dans laquelle le profil de verrouillage (15) et la surface de came (16) d'un bras (11) sont orientés l'un vers l'autre.

5. Pince de préhension (1) selon l'une quelconque des revendications précédentes, dans laquelle au moins un des deux bras (11) comprend une branche (12) et une patte (13) qui s'étend environ parallèlement à la branche (12), la branche (12) et la patte (13) formant entre eux un logement externe (14) ouvert en direction de l'organe de connexion (10), la patte (13) formant la surface de came (16) et la branche (12) formant avantageusement un profil de verrouillage (15) en position fermée.

6. Pince de préhension (1) selon l'une quelconque des revendications précédentes, dans lequel l'organe de connexion (10) comprend des moyens de connexion amovibles (101) adaptés à connecter la pince de préhension (1) de manière amovible à un ustensile de manipulation (3) adapté à saisir la pince (1) et à déplacer ses mâchoires (17) entre les positions fermée et ouverte.

7. Pince de préhension (1) selon l'une quelconque des revendications précédentes, réalisée de manière monobloc, les bras (11) étant reliés à l'organe de connexion (10) par une liaison souple (121).

8. Kit d'implantation comprenant une pince de préhension (1) selon l'une quelconque des revendications précédentes et un implant intervertébral (2) en prise dans la pince de préhension (1).

9. Ensemble d'implantation comprenant une pince de préhension (1) selon l'une quelconque des revendications 1 à 7, un implant intervertébral (2) en prise dans la pince, et un ustensile de manipulation (3) adapté à saisir la pince (1) et à déplacer ses mâchoires (17) entre les positions fermée et ouverte, cet ustensile de manipulation (3) comprenant :
- un tube (31) pourvu d'une poignée (34),
- une tige (37) montée coulissante dans le tube (31) selon un axe de coulissement X, la tige (37) étant connectée à l'organe de connexion (10) de la pince,
- un mécanisme (35) pour déplacer la tige (37) dans le tube (31) selon l'axe de coulissement X,
**caractérisé en ce que** le tube (31) de l'ustensile (3) comprend au moins un organe de contact (33) qui vient en prise avec la surface de came (16) de la pince, lors du coulissement de la tige (37) dans le tube (31).

10. Ensemble d'implantation selon la revendication 9, dans lequel la tige (37) comprend un profil de connexion (38) connecté de manière amovible à l'organe de connexion (10) de la pince, la tige (37) coulisse dans le tube (31) entre une position étendue dans laquelle l'organe de connexion (10) de la pince est engageable sur le profil de connexion (38) de la tige (37) qui fait alors saillie hors du tube (31), et une position rétractée dans laquelle l'organe de contact (33) est en prise avec la surface de came (16) de la pince.

11. Ensemble d'implantation selon la revendication 10, dans lequel la tige (37) est déplaçable dans une position intermédiaire de verrouillage située entre les positions étendue et rétractée, dans laquelle l'organe de contact (33) est alors en prise avec un profil de verrouillage (15) formé par un bras (11) de la pince.

12. Ensemble d'implantation selon la revendication 11, dans lequel l'ustensile (3) comprend des moyens d'indication (36) pour indiquer à l'utilisateur que la tige (37) est en position intermédiaire de verrouillage.

13. Ensemble d'implantation selon l'une quelconque des revendications 9 à 12, dans lequel ledit au moins un organe de contact du tube (31) se présente sous la forme d'une barrette (33) dont la face externe (336) vient en prise avec la surface de came (16) et dont la face interne (335) vient avantageusement en prise avec un profil de verrouillage (15) du bras (11) de la pince.

14. Ensemble d'implantation selon la revendication 10, dans lequel l'organe de connexion (10) de la pince est engagé sur le profil de connexion (38) de l'ustensile de manipulation (3) en position étendue de la tige (37), la connexion entre l'organe de connexion (10) et le profil de connexion (38) étant verrouillée par l'introduction du profil de connexion (38) dans le tube (31), le profil de connexion (38) étant avantageusement déformable élastiquement pour garantir le maintien de l'organe de connexion (10) sur le profil de connexion (38), même avant son introduction dans le tube (31).

## Patentansprüche

1. Zange (1) zum Greifen eines Bandscheibenimplantats (2), enthaltend zwei starre Arme (11), die im Bereich eines Verbindungsglieds (10) gelenkig miteinander verbunden sind, wobei die beiden Arme (11) zwischen sich einen Innenraum (120) definieren, wobei jeder Arm (11) eine Backe (17) bildet, wobei die beiden Backen (17) dazu bestimmt sind, mit einem Bandscheibenimplantat (2) in Eingriff zu gelangen, wobei die Backen (17) zwischen einer Offenstellung und einer Schließstellung in Klemmeingriff mit dem Implantat (2) beweglich sind, wobei zumindest einer der beiden Arme (11) eine Nockenfläche (16) aufweist, um die Backen (17) in die Offenstellung zu beaufschlagen, **dadurch gekennzeichnet, dass** die Nockenfläche (16) sich außerhalb des zwischen den Armen (11) definierten Innenraums (120) erstreckt.

2. Zange (1) nach Anspruch 1, wobei der zumindest eine Arm (11) eine äußere Aufnahmeausnehmung (14) bildet, die sich außerhalb des Innenraums (120) befindet, wobei diese äußere Aufnahmeausnehmung (14) die Nockenfläche (16) bildet.

3. Zange (1) nach Anspruch 1 oder 2, wobei zumindest einer der beiden Arme (11) ein Verriegelungsprofil (15) in Schließstellung enthält.

4. Zange (1) nach Anspruch 3, wobei das Verriegelungsprofil (15) und die Nockenfläche (16) eines Arms (11) aufeinander zugewandt sind.

5. Zange (1) nach einem der vorangehenden Ansprüche, wobei zumindest einer der beiden Arme (11) einen Schenkel (12) und eine Lasche (13) aufweist, die sich in etwa parallel zum Schenkel (12) erstreckt, wobei der Schenkel (12) und die Lasche (13) zwischen sich eine äußere Aufnahmeausnehmung (14) bilden, die in Richtung des Verbindungsglieds (10) offen ist, wobei die Lasche (13) die Nockenfläche(16) bildet und der Schenkel (12) vorteilhaft ein Verriegelungsprofil (15) in Schließstellung bildet.

6. Zange (1) nach einem der vorangehenden Ansprüche, wobei das Verbindungsglied (10) abnehmbare Verbindungsmittel (101) enthält, die dazu geeignet sind, die Zange (1) abnehmbar mit einem Handhabungsutensil (3) zu verbinden, das dazu geeignet ist, die Zange (1) zu greifen und ihre Backen (17) zwischen der Schließ- und der Offenstellung zu verlagern.

7. Zange (1) nach einem der vorangehenden Ansprüche, wobei sie einstückig ausgebildet ist und wobei die Arme (11) über eine nachgiebige Verbindung (121) mit dem Verbindungsglied (10) verbunden sind.

8. Implantationskit mit einer Zange (1) nach einem der vorangehenden Ansprüche und mit einem Bandscheibenimplantat (2), das in der Zange (1) in Eingriff mit dieser ist.

9. Implantationsanordnung mit einer Zange (1) nach einem der Ansprüche 1 bis 7, einem Bandscheibenimplantat (2) in Eingriff mit der Zange und mit einem Handhabungsutensil (3), das dazu geeignet ist, die Zange (1) zu greifen und ihre Backen (17) zwischen der Schließ-und der Offenstellung zu verlagern, wobei dieses Handhabungsutensil (3) aufweist:
- ein Rohr (31), das mit einem Griff (34) versehen ist,
- eine Stange (37), die in einer Verschiebeachse X in dem Rohr (31) verschiebbar gelagert ist, wobei die Stange (37) mit dem Verbindungsglied (10) der Zange verbunden ist,
- einen Mechanismus (35) zum Verlagern der Stange (37) in dem Rohr (31) gemäß der Verschiebeachse X,
**dadurch gekennzeichnet, dass** das Rohr (31) des Utensils (3) zumindest ein Kontaktglied (33) aufweist, das bei der Verschiebung der Stange (37) in dem Rohr (31) mit der Nockenfläche(16) der Zange in Eingriff gelangt.

10. Implantationsanordnung nach Anspruch 9, wobei die Stange (37) ein Verbindungsprofil (38) aufweist, das lösbar mit dem Verbindungsglied (10) der Zange verbunden ist, wobei die Stange (37) in dem Rohr (31) zwischen einer ausgefahrenen Stellung, in welcher das Verbindungsglied (10) der Zange am Verbindungsprofil (38) der Stange (37) angreifen kann, die dann aus dem Rohr (31) vorsteht, und einer eingefahrenen Stellung verschiebbar ist, in welcher das Kontaktglied (33) mit der Nockenfläche (16) der Zange in Eingriff ist.

11. Implantationsanordnung nach Anspruch 10, wobei die Stange (37) in eine Verriegelungszwischenstellung verlagerbar ist, die sich zwischen der ausgefahrenen und der eingefahrenen Stellung befindet, in welcher das Kontaktglied (33) dann mit einem Verriegelungsprofil (15) in Eingriff ist, das von einem Arm (11) der Zange gebildet wird.

12. Implantationsanordnung nach Anspruch 11, wobei das Utensil (3) Anzeigemittel (36) aufweist, um dem Benutzer anzuzeigen, dass die Stange (37) in Verrieglungszwischenstellung ist.

13. Implantationsanordnung nach einem der Ansprüche 9 bis 12, wobei das zumindest eine Kontaktglied des Rohrs (31) in Form eines Stegs (33) vorliegt, dessen Außenseite (336) in Eingriff mit der Nockenfläche (16) gelangt und dessen Innenseite (335) vorteilhaft in Eingriff mit einem Verriegelungsprofil (15) des Arms (11) der Zange gelangt.

14. Implantationsanordnung nach Anspruch 10, wobei das Verbindungsglied (10) der Zange am Verbindungsprofil (38) des Handhabungsutensils (3) in ausgefahrener Stellung der Stange (37) angreift, wobei die Verbindung zwischen dem Verbindungsglied (10) und dem Verbindungsprofil (38) durch Einführen des Verbindungsprofils (38) in das Rohr (31) verriegelt wird, wobei das Verbindungsprofil (38) vorteilhaft elastisch verformt wird, um den Halt des Verbindungsglieds (10) am Verbindungsprofil (38) selbst vor seinem Einführen in das Rohr (31) zu gewährleisten.

## Claims

1. A forceps (1) for gripping an intervertebral implant (2), said forceps comprising two rigid arms (11) that are hinged relative to one another at a connection member (10), the two arms (11) defining between them an internal space (120), each arm (11) forming a jaw (17), the two jaws (17) being for coming into engagement with an intervertebral implant (2), the jaws (17) being movable between an open position and a closed position in clamping engagement with the implant (2), at least one of the two arms (11) including a cam surface (16) for urging the jaws (17) in the open position;
said forceps being **characterized in that** the cam surface (16) extends outside the internal space (120) defined between the arms (11).

2. A forceps (1) according to claim 1, wherein said at least one arm (11) forms an external housing (14) that is situated outside the internal space (120), the external housing (14) forming the cam surface (16).

3. A forceps (1) according to claim 1 or claim 2, wherein at least one of the two arms (11) includes a locking profile (15) for locking in the closed position.

4. A forceps (1) according to claim 3, wherein the locking profile (15) and the cam surface (16) of an arm (11) point towards each other.

5. A forceps (1) according to any preceding claim, wherein at least one of the two arms (11) includes a branch (12) and a tab (13) that extends approximately parallel to the branch (12), the branch (12) and the tab (13) forming between them an external housing (14) that is open towards the connection member (10), the tab (13) forming the cam surface (16), and the branch (12) advantageously forming a locking profile (15) for locking in the closed position.

6. A forceps (1) according to any preceding claim, wherein the connection member (10) includes removable connection means (101) that are adapted to connect the forceps (1) in removable manner to a manipulator tool (3) that is adapted to take hold of the forceps (1) and to move its jaws (17) between the closed and open positions.

7. A forceps (1) according to any preceding claim, made as a single piece, the arms (11) being connected to the connection member (10) via a flexible connection (121).

8. An implantation kit comprising a forceps (1) according to any preceding claim, and an intervertebral implant (2) engaged in the forceps (1).

9. An implantation assembly comprising a forceps (1) according to any one of claims 1 to 3, an intervertebral implant (2) engaged in the forceps, and a manipulator tool (3) that is adapted to take hold of the forceps (1) and to move its jaws (17) between the closed and open positions, the manipulator tool (3) comprising:
• a tube (31) that is provided with a handle (34);
• a rod (37) that is slidably mounted in the tube (31) to slide along a slide axis X, the rod (37) being connected to the connection member (10) of the forceps; and
• a mechanism (35) for moving the rod (37) in the tube (31) along the slide axis X;
the implantation assembly being **characterized in that** the tube (31) of the tool (3) includes at least one contact member (33) that comes into engagement with the cam surface (16) of the forceps while the rod (37) is sliding in the tube (31).

10. An implantation assembly according to claim 9, wherein the rod (37) includes a connection profile (38) that is connected in removable manner to the connection member (10) of the forceps, the rod (37) slides in the tube (31) between an extended position in which the connection member (10) of the forceps is engageable on the connection profile (38) of the rod (37) that thus projects out from the tube (31), and a retracted position in which the contact member (33) is in engagement with the cam surface (16) of the forceps.

11. An implantation assembly according to claim 10, wherein the rod (37) is movable into an intermediate locking position that is situated between the extended and retracted positions, and in which the contact member (33) is thus in engagement with a locking profile (15) that is formed by an arm (11) of the forceps.

12. An implantation assembly according to claim 11, wherein the tool (3) includes indicator means (36) for indicating to the user that the rod (37) is in the intermediate locking position.

13. An implantation assembly according to any one of claims 9 to 12, wherein said at least one contact member of the tube (31) is in the form of a bar (33) having an outer face (336) that comes into engagement with the cam surface (16), and having an inner face (335) that advantageously comes into engagement with a locking profile (15) of the arm (11) of the forceps.

14. An implantation assembly according to claim 10, wherein the connection member (10) of the forceps is engaged on the connection profile (38) of the manipulator tool (3) in the extended position of the rod (37), the connection between the connection member (10) and the connection profile (38) being locked by inserting the connection profile (38) into the tube (31), the connection profile (38) advantageously being elastically deformable so as to guarantee the connection member (10) is held on the connection profile (38), even before it is inserted into the tube (31).
